(19) Europäisches Patentamt
European Patent Office
Office européen des brevets

(11) **EP 1 317 279 B1**

(12) **EUROPEAN PATENT SPECIFICATION**

(45) Date of publication and mention
of the grant of the patent:
**20.07.2011 Bulletin 2011/29**

(51) Int Cl.:
*A61K 39/09* (2006.01)        *A61K 39/385* (2006.01)
*A61P 31/04* (2006.01)

(21) Application number: **01984626.0**

(22) Date of filing: **12.09.2001**

(86) International application number:
**PCT/EP2001/010568**

(87) International publication number:
**WO 2002/022167 (21.03.2002 Gazette 2002/12)**

(54) **VACCINE AGAINST STREPTOCOCCUS PNEUMONIAE**

IMPFSTOFF GEGEN STREPTOCOCCUS PNEUMONIAE

VACCIN CONTRE STREPTOCOCCUS PNEUMONIAE

(84) Designated Contracting States:
**AT BE CH CY DE DK ES FI FR GB GR IE IT LI LU MC NL PT SE TR**
Designated Extension States:
**SI**

(30) Priority: **15.09.2000 GB 0022742**

(43) Date of publication of application:
**11.06.2003 Bulletin 2003/24**

(60) Divisional application:
**10178332.2 / 2 314 313**

(73) Proprietor: **GlaxoSmithKline Biologicals s.a.**
**1330 Rixensart (BE)**

(72) Inventors:
• **LAFERRIERE, Craig Antony Joseph**
**B-1330 Rixensart (BE)**
• **POOLMAN, Jan**
**B-1330 Rixensart (BE)**

(74) Representative: **Lubienski, Michael John**
**GlaxoSmithKline**
**Corporate Intellectual Property**
**CN925.1**
**980 Great West Road**
**Brentford, Middlesex TW8 9GS (GB)**

(56) References cited:
**WO-A-90/06951      WO-A-95/17210**
**WO-A-98/18930      WO-A-99/03884**

• **BRILES D E ET AL: "The potential for using protein vaccines to protect against otitis media caused by Streptococcus pneumoniae" VACCINE, BUTTERWORTH SCIENTIFIC. GUILDFORD, GB, vol. 19, 8 December 2000 (2000-12-08), pages S87-S95, XP004227955 ISSN: 0264-410X**

Remarks:
The file contains technical information submitted after the application was filed and not included in this specification

EP 1 317 279 B1

**Description**

**FIELD OF INVENTION**

The present invention relates to bacterial polysaccharide antigen vaccines, their manufacture and the use of such polysaccharides in medicines.

In particular the present invention relates to vaccines comprising a pneumococcal polysaccharide antigen, typically a pneumococcal polysaccharide conjugate antigen, formulated with a protein antigen from *Streptococcus pneumoniae* and optionally a Th1 inducing adjuvant.

**BACKGROUND OF INVENTION**

*Streptococcus pneumoniae* is a Gram-positive bacteria responsible for considerable morbidity and mortality (particularly in the young and aged), causing invasive diseases such as pneumonia, bacteremia and meningitis, and diseases associated with colonisation, such as acute Otitis media. The rate of pneumococcal pneumonia in the US for persons over 60 years of age is estimated to be 3 to 8 per 100,000. In 20% of cases this leads to bacteremia, and other manifestations such as meningitis, with a mortality rate close to 30% even with antibiotic treatment.

Pneumococcus is encapsulated with a chemically linked polysaccharide which confers serotype specificity. There are 90 known serotypes of pneumococci, and the capsule is the principle virulence determinant for pneumococci, as the capsule not only protects the inner surface of the bacteria from complement, but is itself poorly immunogenic. Polysaccharides are T-independent antigens, and can not be processed or presented on MHC molecules to interact with T-cells. They can however, stimulate the immune system through an alternate mechanism which involves cross-linking of surface receptors on B cells.

It was shown in several experiments that protection against invasive pneumococci disease is correlated most strongly with antibody specific for the capsule, and the protection is serotype specific.

Polysaccharide antigen based vaccines are well known in the art. Four that have been licensed for human use include the Vi polysaccharide of *Salmonella typhi,* the PRP polysaccharide from *Haemophilus influenzae,* the tetravalent meningococcal vaccine composed of serotypes A, C, W135 and Y, and the 23-Valent pneumococcal vaccine composed of the polysaccharides corresponding to serotypes 1, 2, 3, 4, 5, 6B, 7F, 8, 9N, 9V, 10A, 11A, 12F, 14, 15B, 17F, 18C, 19A, 19F, 20, 22F, 23F, and 33 (accounting for at least 90% of pneumococcal blood isolates).

The latter three vaccines confer protection against bacteria causing respiratory infections resulting in severe morbidity and mortality in infants, yet these vaccines have not been licensed for use in children less than two years of age because they are inadequately immunogenic in this age group [Peltola et al.(1984), N. Engl. J. Med. 310:1561-1566]. *Streptococcus pneumoniae* is the most common cause of invasive bacterial disease and otitis media in infants and young children. Likewise, the elderly mount poor responses to pneumococcal vaccines [Roghmann et al., (1987), J. Gerontol. 42:265-270], hence the increased incidence of bacterial pneumonia in this population [Verghese and Berk, (1983) Medicine (Baltimore) 62:271-285].

Strategies, which have been designed to overcome this lack of immunogenicity in infants, include the linking of the polysaccharide to large immunogenic proteins, which provide bystander T-cell help and which induce immunological memory against the polysaccharide antigen to which it is conjugated. Pneumococcal glycoprotein conjugate vaccines are currently being evaluated for safety, immunogenicity and efficacy in various age groups.

The 23-valent unconjugated pneumococcal vaccine has shown a wide variation in clinical efficacy, from 0% to 81% (Fedson et al. (1994) Arch Intern Med. 154: 2531-2535). The efficacy appears to be related to the risk group that is being immunised, such as the elderly, Hodgkin's disease, splenectomy, sickle cell disease and agammaglobulinemics (Fine et al. (1994) Arch Intern Med. 154:2666-2677), and also to the disease manifestation. The 23-valent vaccine does not demonstrate protection against pneumococcal pneumonia (in certain high risk groups such as the elderly) and otitis media diseases.

There is therefore a need for improved pneumococcal vaccine compositions, particularly ones which will be more effective in the prevention or amelioration of pneumococcal disease (particularly pneumonia) in the elderly and in young children.

The present invention provides such an improved vaccine.

**SUMMARY OF THE INVENTION**

Accordingly the present invention provides a vaccine composition, comprising at least one *Streptococcus pneumoniae* polysaccharide antigen (preferably conjugated to a protein carrier) and a *Streptococcus pneumoniae* protein antigen selected from the group consisting of: Poly Histidine Triad family (Pht; e.g. PhtA, PhtB, PhtD, or PhtE), or truncate or immunologically functional equivalent thereof, optionally with a Th1 adjuvant (an adjuvant inducing a predominantly Th1 immune response). Preferably both a pneumococcal protein and Th1 adjuvant are included. Advantageous compositions comprising combinations of the above pneumococcal proteins of the invention with each other and with other pneumococcal proteins are also described. The compositions of the invention are particularly suited in the treatment of elderly pneumonia.

Pneumococcal polysaccharide vaccines (conjugated or not) may not be able to protect against pneumonia in the elderly population for which the incidence of this disease is very high. The key defense mechanism against the pneumococcus is opsonophagocytosis (a humoral B-cell / neutrophil mediated event caused by the production of antibodies against the pneumococcal polysaccharide, the bacterium eventually becoming phagocytosed), however parts of the involved opsonic mechanisms are impaired in the elderly, i.e. superoxide production by PMN (polymorphonuclear cells), other reactive oxygen species production, mobilization of PMN, apoptosis of PMN, deformability of PMN. Antibody responses may also be impaired in the elderly.

Contrary to the normally accepted dogma, normal levels of anti-capsular polysaccharide antibodies may not be effective in complete clearance of bacteria, as pneumococci may invade host cells to evade this branch of the immune system.

Surprisingly, the present inventors have found that by simultaneously stimulating the cell mediated branch of the immune system (for instance T-cell meditated immunity) in addition to the humoral brach of the immune system (B-cell mediated), a synergy (or cooperation) may result which is capable of enhancing the clearance of pneumococci from the host. This is a discovery which will aid the prevention (or treatment) of pneumococcal infection in general, but will be particularly important for the prevention (or treatment) of pneumonia in the elderly where polysaccharide based vaccines do not show efficacy.

Without wishing to be bound by any theory, the present inventors have found that both arms of the immune system may synergise in this way if a pneumococcal polysaccharide (preferably conjugated to a protein carrier) is administered with a pneumococcal protein selected from the group consisting of: PhtA, PhtD, PhtB, and PhtE, (proteins which can be processed and presented in the context of Class II and MHC class I on the surface of infected mammalian cells). Although one or more of these pneumococcal proteins can trigger cell mediated immunity by itself, the inventors have also found that the presence of a Th1 inducing adjuvant in the vaccine formulation helps this arm of the immune system, and surprisingly further enhances the synergy between both arms of the immune system.

**DESCRIPTION OF THE INVENTION**

The present invention provides an improved vaccine particularly for the prevention or amelioration of pnemococcal infection of the elderly (and/or infants and toddlers).

In the context of the invention a patient is considered elderly if they are 55 years or over in age, typically over 60 years and more generally over 65 years.

Thus in one embodiment of the invention there is provided a vaccine composition, suitable for use in the elderly (and/or Infants and toddlers) comprising at least one *Streptococcus pneumoniae* polysaccharide antigen and at least one *Streptococcus pneumoniae* protein antigen(s) selected from the group consisting of: PhtA, PhtD, PhtB, and PhtE, . The vaccine may optionally comprise a Th1 adjuvant.

In a second, preferred, embodiment, the present invention provides a vaccine (suitable for the prevention of pneumonia in the elderly) comprising at least one (2, 3, 4, 5, 6, 7, 8, 9 or 10) *Streptococcus pneumoniae* polysaccharide antigen(s) and at least one *Streptococcus pneumoniae* protein antigen selected from the group consisting of: PhtA, PhtD, PhtB, and PhtE, and, preferably, a Th1 adjuvant.

In the above embodiments vaccines advantageously comprising combinations of the above pneumococcal proteins of the invention with each other and with other pneumococcal proteins are also envisioned as described below.

It is envisaged that such a vaccine will be also useful in treating pneumococcal infection (for instance otitis media) in other high risk groups of the population, such as for infants or toddlers.

*Streptococcus pneumoniae Polysaccharide Antigens of the Invention*

Typically the *Streptococcus pneumoniae* vaccine of the present invention will comprise polysaccharide antigens (preferably conjugated to a carrier protein), wherein the polysaccharides are derived from at least four serotypes of pneumococcus. Preferably the four serotypes include 6B, 14, 19F and 23F. More preferably, at least 7 serotypes are included in the composition, for example those derived from serotypes 4, 6B, 9V, 14, 18C, 19F, and 23F. More preferably still, at least 11 serotypes are included in the composition, for example the composition in one embodiment includes capsular polysaccharides derived from serotypes 1, 3, 4, 5, 6B, 7F, 9V, 14, 18C, 19F and 23F (preferably conjugated to a carrier protein). In a preferred embodiment of the invention at least 13 polysaccharide antigens (preferably conjugated to a carrier protein) are included, although further polysaccharide antigens, for example 23 valent (such as serotypes 1, 2, 3, 4, 5, 6B, 7F, 8, 9N, 9V, 10A, 11A, 12F, 14, 15B, 17F, 18C, 19A, 19F, 20, 22F, 23F and 33F), are also contemplated by the invention.

For elderly vaccination (for instance for the prevention of pneumonia) it is advantageous to include serotypes 8 and 12F (and most preferably 15 and 22 as well) to the 11 valent antigenic composition described above to form a 15 valent vaccine, whereas for infants or toddlers (where otitis media is of more concern) serotypes 6A and 19A are advantageously included to form a 13 valent vaccine.

Although the above polysaccharides may be used in their full-length, native form, it should be understood that size-reduced polysaccharides may also be used which are still immunogenic (see for example EP 497524 and 497525).

For the prevention/amelioration of pneumonia in the elderly (+55 years) population and Otitis media in Infants (up to 18 months) and toddlers (typically 18 months to 5 years), it is a preferred embodiment of the invention to combine a multivalent *Streptococcus pneumonia* polysaccharide as herein described with a *Streraoccus pneumoniae* protein selected from the group consisting of: PhtA, PhtD, PhtB, and PhtE, or immunologically functional equivalent thereof. A combination of pneumococcal proteins may also be advantageously utilised as described below.

*Pneumococcal Proteins of the invention*

For the purposes of this invention, "immunologically functional equivalent" is defined as a peptide of protein comprising at least one protective epitope from the proteins of the invention. Such epitopes are characteristically surface-exposed, highly conserved, and can elicit an bactericidal antibody response in a host or prevent toxic effects. Preferably, the functional equivalent has at least 15 and preferably 30 or more contiguous amino acids from the protein of the invention. Most preferably, fragments, deletions of the protein, such as transmembrane deletion variants thereof (ie the use of the extracellular domain of the proteins), fusions, chemically or genetically detoxified derivatives and the like can be used with the proviso that they are capable of raising substantially the same immune response as the native protein. The position of potential B-cell epitopes in a protein sequence may be readily determined by identifying peptides that are both surface-exposed and antigenic using a combination of two methods: 2D-structure prediction and antigenic index prediction. The 2D-structure prediction can be made using the PSIPRED program (from David Jones, Brunel Bioinformatics Group, Dept. Biological Sciences, Brunel University, Uxbridge UB8 3PH, UK). The antigenic index can be calculated on the basis of the method described by Jameson and Wolf (CABIOS 4:181-186 [1988]).

The proteins of the invention are the following proteins, all of which are exposed on the outer surface of the pneumococcus (capable of being recognised by a host's immune system during at least part of the life cycle of the pneumococcus), or are proteins which are secreted or released by the pneumococcus.

The *Streptococcus pneumoniae* protein of the invention is preferably selected from the group consisting of: a protein from the polyhistidine triad family (Pht), a protein from the Lyt family, a choline binding protein, proteins having an LPXTG motif (where X is any amino acid), proteins having a Type II Signal sequence motif of LXXC (where X is any amino acid), and proteins having a Type I Signal sequence motif. Preferred examples within these categories (or motifs) are the following proteins (or truncate or immunologically functional equivalent thereof):

The Pht (Poly Histidine Triad) family comprises proteins PhtA, PhtB, PhtD, and PhtE. The family is characterised by a lipidation sequence, two domains separated by a proline-rich region and several histidine triads, possibly involved in metal or nucleoside binding or enzymatic activity, (3-5) coiled-coil regions, a conserved N-terminus and a heterogeneous C terminus. It is present in all strains of pneumococci tested. Homologous proteins have also been found in other Streptococci and *Neisseria*. Preferred members of the family comprise PhtA, PhtB and PhtD. More preferably, it comprises PhtA or PhtD. It is understood, however, that the terms Pht A, B, D, and E refer to proteins having sequences disclosed in the citations below as well as naturally-occurring (and man-made) variants thereof that have a sequence homology that is at least 90% identical to the referenced proteins. Preferably it is at least 95% identical and most preferably it is 97% identical.

With regards to the Pht proteins, PhtA is disclosed in WO 98/18930, and is also referred to Sp36. As noted above, it is a protein from the polyhistidine triad family and has the type II signal motif of LXXC.

PhtD is disclosed in WO 00/37105, and is also referred to Sp036D. As noted above, it also is a protein from the polyhistidine triad family and has the type II LXXC signal motif.

PhtB is disclosed in WO 00/37105, and is also referred to Sp036B. Another member of the PhtB family is the C3-Degrading Polypeptide, as disclosed in WO 00/17370. This protein also is from the polyhistidine triad family and has the type II LXXC signal motif. A preferred immunologically functional equivalent is the protein Sp42 disclosed in WO 98/18930. A PhtB truncate (approximately 79kD) is disclosed in WO99115675 which is also considered a member of the PhtX family.

PhtE is disclosed in WO00/30299 and is referred to as BVH-3.

The proteins used in the present invention are preferably selected from the group PhtD and PhtA, or a combination of both of these proteins.

*Advantageous combination of one or more pneumococcal proteins of the invention with other pneumococcal proteins*

In the vaccine of the invention, each of the above proteins of the invention (preferably either or both of PhtD and PhtA) may also be beneficially combined with one or more pneumococcal proteins from the following list: pneumolysin (also referred to as Ply; preferably detoxified by chemical treatment or mutation) [WO 96/05859, WO 90/06951, WO 99/03884], PsaA and transmembrane deletion variants thereof (Berry & Paton, Infect Immun 1996 Dec;64(12):5255-62), PspA and transmembrane deletion variants thereof (US 5804193, WO 92/14488, WO 99/53940), PspC and transmembrane deletion variants thereof (WO 97/09994, WO 99/53940), a member of the Choline binding protein (Cbp) family [e.g. CbpA and transmembrane deletion variants thereof (WO 97/41151; WO 99/51266)], Glyceraldehyde-3-phosphate - dehydrogenase (Infect. Immun. 1996 64:3544), HSP70 (WO 96/40928), PcpA (Sanchez-Beato et al. FEMS Microbiol Lett 1998, 164:207-14), M like protein (SB patent application No. EP 0837130), and adhesion 18627 (SB Patent application No. EP 0834568). The present invention also encompasses immunologically functional equivalents or truncates of such proteins (as defined above).

Concerning the Choline Binding Protein family, members of that family were originally identified as pneumococcal proteins that could be purified by choline-affinity chromatography. All of the choline-binding proteins are non-covalently bound to phosphorylcholine moieties of cell wall teichoic acid and membrane-associated lipoteichoic acid. Structurally, they have several regions in common over the entire family, although the exact nature of the proteins (amino acid sequence, length, etc.) can vary. In general, choline binding proteins comprise an N terminal region (N), conserved repeat regions (R1 and/or R2), a proline rich region (P) and a conserved choline binding region (C), made up of multiple repeats, that comprises approximately one half of the protein. As used in this application, the term "Choline Binding Protein family (Cbp)" is selected from the group consisting of Choline Binding Proteins as identified in WO 97/41151, PbcA, SpsA, PspC, CbpA, CbpD, and CbpG. CbpA is disclosed in WO 97/41151. CbpD and CbpG are disclosed in WO 00/29434. PspC is disclosed in WO 97/09994. PbcA is disclosed in WO 98/21337. Preferably the Choline Binding Proteins are selected from the group consisting of CbpA, PbcA, SpsA and PspC.

If a Cbp is the further protein utilised it may be a Cbp truncate wherein "Cbp" is defined above and "truncate" refers to proteins lacking 50% or more of the Choline binding region (C). Preferably such proteins lack the entire choline binding region. More preferably, the such protein truncates lack (i) the choline binding region and (ii) a portion of the N-terminal half of the protein as well, yet retain at least one repeat region (R1 or R2). More preferably still, the truncate has 2 repeat regions (R1 and R2). Examples of such preferred embodiments are NR1xR2 and R1xR2 as illustrated in WO99/51266 or WO99/51188 however, other choline binding proteins lacking a similar choline binding region are also contemplated within the scope of this invention.

Cbp truncate-Lyt truncate chimeric proteins (or fusions) may also be used in the vaccine of the invention. Preferably this comprises NR1xR2 (or R1xR2) of Cbp and the C-terminal portion (Cterm, i.e., lacking the choline binding domains) of Lyt (e.g., LytCCterm or Sp9lCterm). More preferably Cbp is selected from the group consisting of CbpA, PbcA, SpsA and PspC. More preferably still, it is CbpA. Preferably, Lyt is LytC (also referred to as Sp91).

A PspA or PsaA truncate lacking the choline binding domain (C) and expressed as a fusion protein with Lyt may also be used. Preferably, Lyt is LytC.

*Preferred combinations of pneumococcal proteins for the purposes of this invention*

Preferably the combination of proteins of the invention are selected from 2 or more (3 or 4) different categories such as proteins having a Type II Signal sequence motif of LXXC (where X is any amino acid, e.g., the polyhistidine triad family (Pht)), choline binding proteins (Cbp), proteins having a Type I Signal sequence motif (e.g., Sp101), proteins having a LPXTG motif (where X is any amino acid, e.g., Sp128, Sp130), toxins (e.g., Ply), etc. Preferred examples within these categories (or motifs) are the proteins mentioned above, or immunologically functional equivalents thereof. Toxin + Pht, toxin + Cbp, Pht + Cbp, and toxin + Pht + Cbp are preferred category combinations.

Preferred beneficial combinations include, but are not limited to, PhtD + NR1xR2, PhtD + NR1xR2-Sp91Cterm chimeric or fusion proteins, PhtD + Ply, PhtD + Sp128, PhtD + PsaA, PhtD + PspA, PhtA + NR1xR2, PhtA + NR1xR2-Sp91Cterm chimeric or fusion proteins, PhtA + Ply, PhtA + Sp128, PhtA + PsaA, PhtA + PspA, NR1xR2 + LytC, NR1xR2 + PspA, NR1xR2 + PsaA, NR1xR2 + Sp128, R1xR2 + LytC, R1xR2 + PspA, R1xR2 + PsaA, R1xR2 + Sp128, R1xR2 + PhtD, R1xR2 + PhtA. Preferably, NR1xR2 (or R1xR2) is from CbpA or PspC. More preferably it is from CbpA.

A particularly preferred combination of pneumococcal proteins comprises Ply (or a truncate or immunologically functional equivalent thereof) + PhtD (or a truncate or immunologically functional equivalent thereof) + NR1xR2 (or R1xR2). Preferably, NR1xR2 (or R1xR2) is from CbpA or PspC. More preferably it is from CbpA.

Without wishing to be bound by any theory, within the composition the pneumococcal protein (or combinations described above) of the invention can help to induce a T-cell mediated response against pneumococcal disease - particularly required for protection against pneumonia - which cooperates with the humoral branch of the immune system to inhibit invasion by pneumococci, and to stimulate opsonophagocytosis. A further advantage of including the protein antigen is the presentation of further antigens for the opsonophagocytosis process.

Accordingly in an embodiment of the invention there is provided a *Streptococcus pneumoniae* vaccine comprising a pneumococcus polysaccharide conjugate vaccine comprising polysaccharide antigens derived from at least four serotypes, preferably at least seven serotypes, more preferably at least eleven serotypes, and at least one, but preferably 2, 3, or 4, *Streptococcus pneumoniae* proteins selected from the group consisting of: PhtA, PhtD, PhtB, and PhtE, (or a pneumococcal protein combination as described above). Preferably one of the proteins is PhtA (or an immunologically functional equivalent thereof). Most preferably one of the proteins is PhtD (or an immunologically functional equivalent thereof).

As mentioned above, a problem associated with the polysaccharide approach to vaccination, is the fact that polysaccharides *per se* are poor immunogens. To overcome this, polysaccharides may be conjugated to protein carriers, which provide bystander T-cell help. It is preferred, therefore, that the polysaccharides utilised in the invention are linked to such a protein carrier. Examples of such carriers which are currently commonly used for the production of polysaccharide immunogens include the Diphtheria and Tetanus toxoids (DT, DT CRM197 and TT respectively), Keyhole Limpet Haemocyanin (KLH), OMPC from *N. meningitidis.* and the purified protein derivative of Tuberculin (PPD).

A preferred carrier for the pneumococcal polysaccharide based immunogenic compositions (or vaccines) is protein D from *Haemophilus influenza* (EP 594610-B), or fragments thereof. Fragments suitable for use include fragments encompassing T-helper epitopes. In particular a protein D fragment will preferably contain the N-terminal 1/3 of the protein. A protein D carrier is surprisingly useful as a carrier in vaccines where multiple pneumococcal polysaccharide antigens are conjugated. Epitope suppression is usually likely to occur if the same carrier is used for each polysaccharide. Surprisingly, the present inventors have found protein D is particularly suitable for minimising such epitopic suppression effects in combination vaccines. One or more pneumococcal polysaccharides in a combination may be advantageously conjugated onto protein D, and preferably all antigens are conjugated onto protein D within such a combination vaccine.

A further preferred carrier for the pneumococcal polysaccharide is the pneumococcal protein itself (as defined above in section "Pneumococcal Proteins of the invention").

The polysaccharide may be linked to the carrier protein by any known method (for example, by Likhite, U.S. Patent 4,372,945 and by Armor et al., U.S. Patent 4,474,757). Preferably, CDAP conjugation is carried out (WO 95/08348).

Preferably the protein:polysaccharide (weight:weight) ratio of the conjugates is 0.3:1 to 1:1, more preferably 0.6:1 to 0.8:1, and most preferably about 0.7:1.

The vaccines of the present invention are preferably adjuvanted. Suitable adjuvants include an aluminium salt such as aluminium hydroxide gel (alum) or aluminium phosphate, but may also be a salt of calcium, magnesium, iron or zinc, or may be an insoluble suspension of acylated tyrosine, or acylated sugars, cationically or anionically derivatised polysaccharides, or polyphosphazenes.

It is preferred that the adjuvant be selected to be a preferential inducer of a TH1 type of response to aid the cell mediated branch of the immune response.

*TH1 Adjuvants of the Invention*

High levels of Th1-type cytokines tend to favour the induction of cell mediated immune responses to a given antigen, whilst high levels of Th2-type cytokines tend to favour the induction of humoral immune responses to the antigen.

It is important to remember that the distinction of Th1 and Th2-type immune response is not absolute. In reality an individual will support an immune response which is described as being predominantly Th1 or predominantly Th2. However, it is often convenient to consider the families of cytokines in terms of that described in murine CD4 +ve T cell clones by Mosmann and Coffman (Mosmann, T.R. and Coffman, R.L. (1989) TH1 and TH2 cells: different patterns of lymphokine secretion lead to different functional properties. Annual Review of Immunology, 7, p145-173). Traditionally, Th1-type responses are associated with the production of the INF-γ and IL-2 cytokines by T-lymphocytes. Other cytokines often directly associated with the induction of Th1-type immune responses are not produced by T-cells, such as IL-12. In contrast, Th2-type responses are associated with the secretion of Il-4, IL-5, IL-6, IL-10. Suitable adjuvant systems which promote a predominantly Th1 response include: Monophosphoryl lipid A or a derivative thereof, particularly 3-de-O-acylated monophosphoryl lipid A (3D-MPL) (for its preparation see GB 2220211 A); and a combination of monophosphoryl lipid A, preferably 3-de-O-acylated monophosphoryl lipid A, together with either an aluminium salt (for instance aluminium phosphate or aluminium hydroxide) or an oil-in-water emulsion. In such combinations, antigen and 3D-MPL are contained in the same particulate structures, allowing for more efficient delivery of antigenic and immunostimulatory signals. Studies have shown that 3D-MPL is able to further enhance the immunogenicity of an alum-adsorbed antigen [Thoelen et al. Vaccine (1998) 16:708-14; EP 689454-B1].

An enhanced system involves the combination of a monophosphoryl lipid A and a saponin derivative, particularly the combination of QS21 and 3D-MPL as disclosed in WO 94/00153, or a less reactogenic composition where the QS21 is quenched with cholesterol as disclosed in WO 96/33739.

A particularly potent adjuvant formulation involving QS21, 3D-MPL and tocopherol in an oil in water emulsion is described in WO 95/17210, and is a preferred formulation.

Preferably the vaccine additionally comprises a saponin, more preferably QS21. The formulation may also comprises an oil in water emulsion and tocopherol (WO 95/17210).

The present invention also provides a method for producing a vaccine formulation comprising mixing a protein of the present invention together with a pharmaceutically acceptable excipient, such as 3D-MPL.

Unmethylated CpG containing oligonucleotides (WO 96/02555) are also preferential inducers of a TH1 response and are suitable for use in the present invention.

Particularly preferred compositions of the invention comprise one or more conjugated pneumococcal polysaccharides, one or more pneumococcal proteins of the invention and a Th1 adjuvant. Without wishing to be bound by any theory, the induction of a cell mediated response by way of a pneumococcal protein (as described above) and the cooperation between both arms of the immune system may be aided using such a Th-1 adjuvant, resulting in a particularly effective vaccine against pneumococcal disease in general, and, importantly, against pneumococcal pneumonia in the elderly.

In a further aspect of the present invention there is provided an immunogen or vaccine as herein described for use in medicine.

In one embodiment there is a method of preventing or ameliorating pneumonia in an elderly human (+55 years) comprising administering a safe and effective amount of a vaccine, as described herein, comprising a *Streptoccocus pneumoniae* polysaccharide antigen and a pneumococcal protein selected from the group consisting of PhtA, PhtD, PhtB, and PhtE, and optionally a Th1 adjuvant, to said elderly patient.

In a further embodiment there is provided a method of preventing or ameliorating otitis media in Infants (up to 18 months) or toddlers (typically 18 months to 5 years), comprising administering a safe and effective amount of a vaccine comprising a *Streptococcus pneumoniae* polysaccharide antigen and a *Streptococcus pneumoniae* protein antigen selected from the group consisting of: PhtA, PhtD, PhtB, and PhtE, and optionally a Th1 adjuvant, to said Infant or toddler.

Preferably in the methods of the invention as described above the polysaccharide antigen is present as a polysaccharide protein conjugate.

*Vaccine Preparations of the Invention*

The vaccine preparations of the present invention may be used to protect or treat a mammal susceptible to infection, by means of administering said vaccine via systemic or mucosal route. These administrations may include injection via the intramuscular, intraperitoneal, intradermal or subcutaneous routes; or via mucosal administration to the oral/alimentary, respiratory, genitourinary tracts. Intranasal administration of vaccines for the treatment of pneumonia or otitis media is preferred (as nasopharyngeal carriage of pneumococci can be more effectively prevented, thus attenuating infection at its earliest stage). Although the vaccine of the invention may be administered as a single dose, components thereof may also be co-administered together at the same time or at different times (for instance pneumococcal polysaccharides could be administered separately at the same time or 1-2 weeks after the administration of the bacterial protein component of the vaccine for optimal coordination of the immune responses with respect to each other). For coadministration, the optional Th1 adjuvant may be present in any or all of the different administrations, however it is preferred if it is present in combination with the bacterial protein component of the vaccine. In addition to a single route of administration, 2 different routes of administration may be used. For example, any viral antigens may be administered ID (intradermal), whilst bacterial proteins may be administered IM (intramuscular) or IN (intranasal). Polysaccharides may be administered IM (or ID) and bacterial proteins may be administered IN (or ID). In addition, the vaccines of the invention may be administered IM for priming doses and IN for booster doses.

The amount of conjugate antigen in each vaccine dose is selected as an amount which induces an immunoprotective response without significant, adverse side effects in typical vaccines. Such amount will vary depending upon which specific immunogen is employed and how it is presented. Generally, it is expected that each dose will comprise 0.1-100 $\mu$g of polysaccharide, preferably 0.1-50 $\mu$g, preferably 0.1-10 $\mu$g, of which 1 to 5 $\mu$g is the most preferable range.

The content of protein antigens in the vaccine will typically be in the range 1-100$\mu$g, preferably 5-50$\mu$g, most typically in the range 5 - 25$\mu$g.

Optimal amounts of components for a particular vaccine can be ascertained by standard studies involving observation of appropriate immune responses in subjects. Following an initial vaccination, subjects may receive one or several booster immunisations adequately spaced.

Vaccine preparation is generally described in Vaccine Design ("The subunit and adjuvant approach" (eds Powell M.F. & Newman M.J.) (1995) Plenum Press New York). Encapsulation within liposomes is described by Fullerton, US Patent 4,235,877.

Although the vaccines of the present invention may be administered by any route, administration of the described vaccines into the skin (ID) forms one embodiment of the present invention. Human skin comprises an outer "horny" cuticle, called the stratum corneum, which overlays the epidermis. Underneath this epidermis is a layer called the dermis, which in turn overlays the subcutaneous tissue. Researchers have shown that injection of a vaccine into the skin, and in particular the dermis, stimulates an immune response, which may also be associated with a number of additional advantages. Intradermal vaccination with the vaccines described herein forms a preferred feature of the present invention.

The conventional technique of intradermal injection, the "mantoux procedure", comprises steps of cleaning the skin, and then stretching with one hand, and with the bevel of a narrow gauge needle (26-31 gauge) facing upwards the needle is inserted at an angle of between 10-15°. Once the bevel of the needle is inserted, the barrel of the needle is lowered and further advanced whilst providing a slight pressure to elevate it under the skin. The liquid is then injected very slowly thereby forming a bleb or bump on the skin surface, followed by slow withdrawal of the needle.

More recently, devices that are specifically designed to administer liquid agents into or across the skin have been described, for example the devices described in WO 99/34850 and EP 1092444, also the jet injection devices described for example in WO 01/13977; US 5,480,381, US 5,599,302, US 5,334,144, US 5,993,412, US 5,649,912, US 5,569,189, US 5,704,911, US 5,383,851, US 5,893,397, US 5,466,220, US 5,339,163, US 5,312,335, US 5,503,627, US 5,064,413, US 5,520, 639, US 4,596,556, US 4,790,824, US 4,941,880, US 4,940,460, WO 97137705 and WO 97/13537. Alternative methods of intradermal administration of the vaccine preparations may include conventional syringes and needles, or devices designed for ballistic delivery of solid vaccines (WO 99/27961), or transdermal patches (WO 97/48440; WO 98/28037); or applied to the surface of the skin (transdermal or transcutaneous delivery WO 98/20734 ; WO 98/28037).

When the vaccines of the present invention are to be administered to the skin, or more specifically into the dermis, the vaccine is in a low liquid volume, particularly a volume of between about 0.05 ml and 0.2 ml.

The content of antigens in the skin or intradermal vaccines of the present invention may be similar to conventional doses as found in intramuscular vaccines (see above). However, it is a feature of skin or intradermal vaccines that the formulations may be "low dose". Accordingly the protein antigens in "low dose" vaccines are preferably present in as little as 0.1 to 10μg, preferably 0.1 to 5 μg per dose; and the polysaccharide (preferably conjugated) antigens may be present in the range of 0. 1-1μg, and preferably between 0.01 to 0.5 μpg of polysaccharide per dose.

As used herein, the term "intradermal delivery" means delivery of the vaccine to the region of the dermis in the skin. However, the vaccine will not necessarily be located exclusively in the dermis. The dermis is the layer in the skin located between about 1.0 and about 2.0 mm from the surface in human skin, but there is a certain amount of variation between individuals and in different parts of the body. In general, it can be expected to reach the dermis by going 1.5 mm below the surface of the skin. The dermis is located between the stratum corneum and the epidermis at the surface and the subcutaneous layer below. Depending on the mode of delivery, the vaccine may ultimately be located solely or primarily within the dermis, or it may ultimately be distributed within the epidermis and the dermis.

The present invention also contemplates combination vaccines which provide protection against a range of different pathogens. Many Paediatric vaccines are now given as a combination vaccine so as to reduce the number of injections a child has to receive. Thus for Paediatric vaccines other antigens from other pathogens may be formulated with the vaccines of the invention. For example the vaccines of the invention can be formulated with (or administered separately but at the same time) the well known 'trivalent' combination vaccine comprising Diphtheria toxoid (DT), tetanus toxoid (TT), and pertussis components [typically detoxified Pertussis toxoid (PT) and filamentous haemagglutinin (FHA) with optional pertactin (PRN) and/or agglutinin 1+2], for example the marketed vaccine INFANRIX-DTPa™ (SmithKlineBeecham Biological) which contains DT, TT, PT, FHA and PRN antigens, or with a whole cell pertussis component for example as marketed by SmithKlineBeecham Biologicals s.a., as Tritanrix™. The combined vaccine may also comprise other antigen, such as Hepatitis B surface antigen (HBsAg), Polio virus antigens (for instance inactivated trivalent polio virus - IPV), *Moraxella catarrhalis* outer membrane proteins, non-typeable *Haemophilus influenza* proteins, *N. meningitidis* B outer membrane proteins.

Examples of preferred *Moraxella catarrhalis* protein antigens which can be included in a combination vaccine (especially for the prevention of otitis media) are: OMP106 [WO 97/41731 (Antex) & WO 96/34960 (PMC)]; OMP21; LbpA &/or LbpB [WO 98/55606 (PMC)]; TbpA &/or TbpB [WO 97/13785 & WO 97/32980 (PMC)]; CopB [Helminen ME, et al. (1993) Infect. Immun. 61:2003-2010]; UspA1 and/or UspA2 [WO 93/03761 (University of Texas)]; OmpCD; HasR (PCT/EP99/03824); PilQ (PCT/EP99/03823); OMP85 (PCT/EP00/01468); lipo06 (GB 9917977.2); lipo10 (GB 9918208.1); lipo11 (GB 9918302.2); lipo18 (GB 9918038.2); P6 (PCT/EP99/03038); D15 (PCT/EP99/03822); Omp1A1 (PCT/EP99/06781); Hly3 (PCT/EP99/03257); and OmpE. Examples of non-typeable *Haemophilus influenzae* antigens which can be included in a combination vaccine (especially for the prevention of otitis media) include: Fimbrin protein [(US 5766608 - Ohio State Research Foundation)] and fusions comprising peptides therefrom [eg LB1 (f) peptide fusions; US 5843464 (OSU) or WO 99/64067]; OMP26 [WO 97/01638 (Cortecs)]; P6 [EP 281673 (State University of New York)]; TbpA and/or TbpB; Hia; Hsf; Hin47; Hif; Hmw1; Hmw2; Hmw3; Hmw4; Hap; D15 (WO 94/12641); protein D (EP 594610); P2; and P5 (WO 94/26304).

Other combinations contemplated are the pneumococcal PS & protein of the invention in combination with viral antigens, for example, from influenza (attenuated, split, or subunit [e.g., surface glycoproteins neuraminidase (NA) and haemagglutinin (HA). See, e.g., Chaloupka I. et al, Eur. Journal Clin. Microbiol. Infect. Dis. 1996, 15:121-127], RSV (e.g., F and G antigens or F/G fusions, see, eg, Schmidt A. C. et al, J Virol, May 2001, p4594 - 4603), PIV3 (e.g., HN and F proteins, see Schmidt et al. supra), Varicella (e.g., attenuated, glycoproteins I-V, etc.), and any (or all) component (s) of MMR (measles, mumps, rubella).

A preferred Peadiatric combination vaccine contemplated by the present invention for global treatment or prevention of otitis media comprises: one or more *Streptococcus pneumoniae* polysaccharide antigen(s) (preferably conjugated to protein D), one or more pneumococcal proteins selected from the group consisting of: PhtA, PhtD, PhtB, and PhtE, (or an immunologically functional equivalent thereof, and one or more surface-exposed antigen from *Moraxella catarrhalis* and/or non-typeable *Haemophilus influenzae.* Protein D can advantageously be used as a protein carrier for the pneumococcal polysaccharides to overcome epitope suppression problems (as mentioned above), and because it is in itself an immunogen capable of producing B-cell mediated protection against non-typeable *H. influenzae* (ntHi). The *Moraxella catarrhalis* or non-typeable *Haemophilus influenzae* antigens can be included in the vaccine in a sub-unit form, or may be added as antigens present on the surface of outer membrane vesicles (blebs) made from the bacteria.

Preferably the antigenic compositions (and vaccines) hereinbefore described are lyophilised up until they are about to be used, at which point they are extemporaneously reconstituted with diluent. More preferably they are lyophilised in the presence of 3D-MPL, and are extemporaneously reconstituted with saline solution. Alternatively, the protein and polysaccharide may be stored separately in a vaccination kit (either or both components being lyophilised), the components being reconstituted and either mixed prior to use or administered separately to the patient. A Th1 adjuvant (preferably 3D-MPL) may be present with either or both of the components.

The lyophilisation of vaccines is well known in the art. Typically the liquid vaccine is freeze dried in the presence of an anti-caking agent for instance sugars such as sucrose or lactose (present at an initial concentration of 10-200 mg/mL). Lyophilisation typically occurs over a series of steps, for instance a cycle starting at - 69 °C, gradually adjusting to -24 °C over 3 hours, then retaining this temperature for 18 hours, then gradually adjusting to -16 °C over 1 hour, then retaining this temperature for 6 hours, then gradually adjusting to +34 °C over 3 hours, and finally retaining this temperature over 9 hours.

Lyophilising the compositions results in a more stable composition (for instance it prevents the breakdown of the polysaccharide antigens). The process is also surprisingly responsible for a higher antibody titre against the pneumococcal polysaccharides. This has been shown to be particularly significant for PS 6B conjugates. Another aspect of the invention is thus a lyophilised antigenic composition comprising a PS 6B conjugate adjuvanted with 3D-MPL (preferably devoid of aluminium-based adjuvants) and a pneumococcal protein selected from the group consisting of: PhtA, PhtD, PhtB, and PhtE, .

## EXAMPLES

The examples illustrate, but do not limit the invention.

### Example 1

*S.pneumonlae capsular polysaccharide:*

The 11-valent candidate vaccine includes the capsular polysaccharides serotypes 1, 3, 4, 5, 6B, 7F, 9V, 14, 18C, 19F and 23F which were made essentially as described in EP 72513. Each polysaccharide is activated and derivatised using CDAP chemistry (WO 95/08348) and conjugated to the protein carrier. All the polysaccharides are conjugated in their native form, except for the serotype 3 (which was size-reduced to decrease its viscosity).

*Protein carrier:*

The protein carrier selected is the recombinant protein D (PD) from Non typeable *Haemophilus influenzae,* expressed in *E. coli.*

## EXPRESSION OF PROTEIN D

### *Haemophilus influenzae* protein D

### Genetic construction for protein D expression

### Starting materials

### *The Protein D encoding DNA*

Protein D is highly conserved among *H. influenza* of all serotypes and non-typeable strains. The vector pHIC348 containing the DNA sequence encoding the entire protein D gene has been obtained from Dr. A. Forsgren, Department of Medical Microbiology, University of Lund, Malmö General Hospital, Malmö, Sweden. The DNA sequence of protein D has been published by Janson et al. (1991) Infect. Immun. 59: 119-125.

### *The expression vector pMG1*

The expression vector pMG1 is a derivative of pBR322 (Gross *et al.,* 1985) in which bacteriophage λ derived control elements for transcription and translation of foreign inserted genes were introduced (Shatzman *et al.,* 1983). In addition, the Ampicillin resistance gene was exchanged with the Kanamycin resistance gene.

### *The E. coli strain AR58*

The *E. coli* strain AR58 was generated by transduction of N99 with a P1 phage stock previously grown on an SA500 derivative (galE::TN10, lambdaKil⁻ cI857 ΔH1). N99 and SA500 are *E. coli* K12 strains derived from Dr. Martin Rosenberg's laboratory at the National Institute of Health.

### *The expression vector pMG 1*

For the production of protein D, the DNA encoding the protein has been cloned into the expression vector pMG 1. This plasmid utilises signals from lambdaphage DNA to drive the transcription and translation of inserted foreign genes. The vector contains the promoter PL, operator OL and two utilisation sites (NutL and NutR) to relieve transcriptional polarity effects when N protein is provided (Gross *et al.,* 1985). Vectors containing the PL promoter, are introduced into an *E. coli* lysogenic host to stabilise the plasmid DNA. Lysogenic host strains contain replication-defective lambdaphage DNA integrated into the genome (Shatzman *et al.,* 1983). The chromosomal lambdaphage DNA directs the synthesis of the cI repressor protein which binds to the OL repressor of the vector and prevents binding of RNA polymerase to the PL promoter and thereby transcription of the inserted gene. The cI gene of the expression strain AR58 contains a temperature sensitive mutant so that PL directed transcription can be regulated by temperature shift, i.e. an increase in culture temperature inactivates the repressor and synthesis of the foreign protein is initiated. This expression system allows controlled synthesis of foreign proteins especially of those that may be toxic to the cell (Shimataka & Rosenberg, 1981).

### *The E. coli strain AR58*

The AR58 lysogenic *E. coli* strain used for the production of the protein D carrier is a derivative of the standard NIH E. *coli* K12 strain N99 (F⁻ su⁻ galK2, lacZ⁻ thr⁻). It contains a defective lysogenic lambdaphage (galE::TN10, lambdaKil⁻ cI857 ΔH1). The Kil⁻phenotype prevents the shut off of host macromolecular synthesis. The cI857 mutation confers a temperature sensitive lesion to the cI repressor. The ΔH1 deletion removes the lambdaphage right operon and the hosts bio, uvr3, and ch1A loci. The AR58 strain was generated by transduction of N99 with a P1 phage stock previously grown on an SA500 derivative (galE::TN10, lambdaKil⁻ cI857 ΔH1). The introduction of the defective lysogen into N99 was selected with tetracycline by virtue of the presence of a TN10 transposon coding for tetracyclin resistance in the adjacent galE gene.

### *Construction of vector pMGMDPPrD*

The pMG 1 vector which contains the gene encoding the non-structural S 1 protein of Influenzae virus (pMGNSI) was used to construct pMGMDPPrD. The protein D gene was amplified by PCR from the pHIC348 vector (Janson *et al.* 1991) with PCR primers containing NcoI and XbaI restriction sites at the 5' and 3' ends, respectively. The NcoI/XbaI fragment was then introduced into pMGNS1 between NcoI and XbaI thus creating a fusion protein containing the N-terminal 81 amino acids of the NS1 protein followed by the PD protein. This vector was labeled pMGNS1PrD.

Based on the construct described above the final construct for protein D expression was generated. A BamHI/ BamHI fragment was removed from pMGNS1PrD. This DNA hydrolysis removes the NS1 coding region, except for the first three N-terminal residues. Upon religation of the vector a gene encoding a fusion protein with the following N-terminal amino acid sequence has been generated:

----MDP SSHSSNMANT-----

NS1                 Protein D

.

The protein D does not contain a leader peptide or the N-terminal cysteine to which lipid chains are normally attached. The protein is therefore neither excreted into the periplasm nor lipidated and remains in the cytoplasm in a soluble form.

The final construct pMG-MDPPrD was introduced into the AR58 host strain by heat shock at 37 °C. Plasmid containing bacteria were selected in the presence of Kanamycin. Presence of the protein D encoding DNA insert was demonstrated by digestion of isolated plasmid DNA with selected endonucleases. The recombinant E. *coli* strain is referred to as ECD4.

Expression of protein D is under the control of the lambda $P_L$ promoter/ $O_L$ Operator. The host strain AR58 contains a temperature-sensitive cI gene in the genome which blocks expression from lambda $P_L$ at low temperature by binding to $O_L$. Once the temperature is elevated cI is released from $O_L$ and protein D is expressed. At the end of the fermentation the cells are concentrated and frozen.

The extraction from harvested cells and the purification of protein D was performed as follows. The frozen cell culture pellet is thawed and resuspended in a cell disruption solution (Citrate buffer pH 6.0) to a final $OD_{650}$ = 60. The suspension is passed twice through a high pressure homogenizer at P = 1000 bar. The cell culture homogenate is clarified by centrifugation and cell debris are removed by filtration. In the first purification step the filtered lysate is applied to a cation exchange chromatography column (SP Sepharose Fast Flow). PD binds to the gel matrix by ionic interaction and is eluted by a step increase of the ionic strength of the elution buffer.

In a second purification step impurities are retained on an anionic exchange matrix (Q Sepharose Fast Flow). PD does not bind onto the gel and can be collected in the flow through.

In both column chromatography steps fraction collection is monitored by OD. The flow through of the anionic exchange column chromatography containing the purified protein D is concentrated by ultrafiltration.

The protein D containing ultrafiltration retentate is finally passed through a 0.2 $\mu$m membrane.

*Chemistry:*

*Activation and coupling chemistry:*

The activation and coupling conditions are specific for each polysaccharide. These are given in Table 1. Native polysaccharide (except for PS3) was dissolved in NaCl 2M or in water for injection. The optimal polysaccharide concentration was evaluated for all the serotypes.

From a 100 mg/ml stock solution in acetonitrile, CDAP (CDAP/PS ratio 0.75 mg/mg PS) was added to the polysaccharide solution. 1.5 minute later, 0.2M triethylamine was added to obtain the specific activation pH. The activation of the polysaccharide was performed at this pH during 2 minutes at 25 °C. Protein D (the quantity depends on the initial PS/PD ratio) was added to the activated polysaccharide and the coupling reaction was performed at the specific pH for 1 hour. The reaction was then quenched with glycine for 30 minutes at 25 °C and overnight at 4 °C.

The conjugates were purified by gel filtration using a Sephacryl 500HR gel filtration column equilibrated with 0.2M NaCl.

The carbohydrate and protein content of the eluted fractions was determined. The conjugates were pooled and sterile filtered on a 0.22$\mu$m sterilizing membrane. The PS/Protein ratios in the conjugate preparations were determined.

*Characterisation:*

Each conjugate was characterised and met the specifications described in Table 2. The polysaccharide content ($\mu$g/ml) was measured by the Resorcinol test and the protein content ($\mu$g/ml) by the Lowry test. The final PS/PD ratio (w/w) is determined by the ratio of the concentrations.

*Residual DMAP content (ng/μg PS):*

The activation of the polysaccharide with CDAP introduces a cyanate group in the polysaccharide and DMAP (4-dimethylamino-pyridin) is liberated. The residual DMAP content was determined by a specific assay developed at SB.

**Free polysaccharide content (%):**

The free polysaccharide content of conjugates kept at 4°C or stored 7 days at 37°C was determined on the supernatant obtained after incubation with α-PD antibodies and saturated ammonium sulfate, followed by a centrifugation.
An α-PS/α-PS ELISA was used for the quantification of free polysaccharide in the supernatant . The absence of conjugate was also controlled by an α-PD/α-PS ELISA. Reducing the quantity of free polysaccharide results in an improved conjugate vaccine.

**Antigenicity:**

The antigenicity on the same conjugates was analyzed in a sandwich-type ELISA wherein the capture and the detection of antibodies were α-PS and α-PD respectively.

**Free protein content (%):**

The level of "free" residual protein D was determined by using a method with SDS treatment of the sample. The conjugate was heated 10 min at 100°C in presence of SDS 0.1 % and injected on a SEC-HPLC gel filtration column (TSK 3000-PWXL). As protein D is dimer, there is a risk of overestimating the level of "free" protein D by dissociation the structure with SDS.

**Molecular size ($K_{av}$):**

The molecular size was performed on a SEC-HPLC gel filtration column (TSK 5000-PWXL).

**Stability:**

The stability was measured on a HPLC-SEC gel filtration (TSK 6000-PWXL) for conjugates kept at 4°C and stored for 7 days at 37°C.
The 11-valent characterization is given in Table 2
The protein conjugates can be adsorbed onto aluminium phosphate and pooled to form the final vaccine.

**Conclusion:**

Immunogenic conjugates have been produced, that have since been shown to be components of a promising vaccine. The optimised CDAP conditions for the best quality final conjugated pneumococcal polysaccharide product was discovered for each of the 11 valencies.

<u>Table 1</u>
<u>Specific activation/coupling/quenching conditions of PS S.pneumoniae-Protein D</u>
<u>conjugates</u>

| Serotype | 1 | 3 (μfluid.) | 4 | 5 | 6B | 7F |
|---|---|---|---|---|---|---|
| PS conc.(mg/ml) | 2.0 | 3.0 | 2.0 | 7.5 | 5.4 | 3.0 |
| PS dissolution | NaCl 2M | NaCl 2M | $H_2O$ | $H_2O$ | NaCl 2M | NaCl 2M |
| PD conc.(mg/ml) | 5.0 | 5.0 | 5.0 | 5.0 | 5.0 | 5.0 |
| Initial PS/PD Ratio (w/w) | 1/1 | 1/1 | 1/1 | 1/1 | 1/1 | 1/1 |
| CDAP conc. (mg/mg PS) | 0.75 | 0.75 | 0.75 | 0.75 | 0.75 | 0.75 |
| $pH_a=pH_c=pH_q$ | 9.0/9.0/9.0 | 9.0/9.0/9.0 | 9.0/9.0/9.0 | 9.0/9.0/9.0 | 9.5/9.5/9.0 | 9.0/9.0/9.0 |

| Serotype | 9V | 14 | 18C | 19F | 23F |
|---|---|---|---|---|---|
| PS conc.(mg/ml) | 2.5 | 2.5 | 2.0 | 4.0 | 3.3 |
| PS dissolution | NaCl 2M | NaCl 2M | $H_2O$ | NaCl 2M | NaCl 2M |
| PD conc.(mg/ml) | 5.0 | 5.0 | 5.0 | 5.0 | 5.0 |
| Initial PS/PD Ratio (w/w) | 1/0.75 | 1/0.75 | 1/1 | 1/0.5 | 1/1 |
| CDAP conc. (mg/mg PS) | 0.75 | 0.75 | 0.75 | 0.75 | 0.75 |
| $pH_a=pH_c=pH_q$ | 8.5/8.5/9.0 | 9.0/9.0/9.0 | 9.0/9.0/9.0 | 10/9.5/9.0 | 9.0/9.0/9.0 |

TABLE 2: Specifications of the 11 valent pneumococcoal PS-PD vaccine (first numbers of the batch code indicates serotype)

| Criteria | D01PDJ227 | D03PDJ236 | D4PDJ228 | D5PDJ235 | D6PDJ209 | |
|---|---|---|---|---|---|---|
| Ratio PS/ Prot (w/w) | 1/0.66 | 1/1.09 | 1/0.86 | 1/0.86 | 1/0.69 | |

(continued)

| Criteria | D01PDJ227 | D03PDJ236 | D4PDJ228 | D5PDJ235 | D6PDJ209 | |
|---|---|---|---|---|---|---|
| Free polysac. content (%) <10 % | 1 | 1 | 7 | 9 | 0 | |
| Free protein content (%) <15 % | 8 | <1 | 19 | 21 | 9 | |
| DMAP content (ng/ $\mu$g PS) < 0.5 ng/$\mu$g PS | 0.2 | 0.6 | 0.4 | 1.2 | 0.3 | |
| Molecular size ($K_{av}$) | 0.18 | 0.13 | 0.12 | 0.11 | 0.13 | |
| Stability | no shift | no shift | no shift | low shift | no shift | |
| | D07PDJ225 | D09PDJ222 | D14PDJ202 | D18PDJ221 | D19PDJ206 | D23PDJ212 |
| Ratio PS/ Prot (w/w) | 1/0.58 | 1/0.80 | 1/0.68 | 1/0.62 | 1/0.45 | 1/0.74 |
| Free polysac. content (%) <10 % | 1 | <1 | <1 | 4 | 4 | 0 |
| Free protein content (%) <15 % | 8 | 0.3 | 3 | 21 | 10 | 12 |
| DMAP content (ng/ $\mu$g PS) <0.5 ng/pg PS | 0.1 | 0.6 | 0.3 | 0.2 | 0.1 | 0.9 |
| Molecular size ($K_{av}$) | 0.14 | 0.14 | 0.17 | 0.10 | 0.12 | 0.12 |
| Stability | no shift | no shift | no shift | no shift | shift | no shift |

**Example 2 - Beneficial impact of the addition of one or more of the pneumococcal proteins of the invention +/- 3D-MPL on the protective effectiveness of PD-conjugated 11-valent polysaccharide vaccine against pneumococcal lung colonization in mice**

***Immunological read-outs***

*ELISA dosage of pneumococcal protein-specific serum IgG*

Maxisorp Nunc immunoplates are coated for 2 hours at 37°C with 100 $\mu$l/well of 2 $\mu$g/ml protein diluted in PBS. Plates are washed 3 times with NaCl 0.9% Tween-20 0.05% buffer. Then, serial 2-fold dilutions (in PBS/ Tween-20 0.05%, 100 $\mu$l per well) of an anti-protein serum reference added as a standard curve (starting at 670 ng/ml IgG) and serum samples (starting at a 1/10 dilution) are incubated for 30 minutes at 20°C under agitation. After washing as previously described, peroxydase-conjugated goat anti-mouse IgG (Jackson) diluted 5000x in PBS/ Tween-20 0.05% are incubated (100 $\mu$l/well) for 30 minutes at 20°C under agitation. After washing, plates are incubated for 15 min at room temperature with 100 $\mu$l/well of revelation buffer (OPDA 0.4 mg/ml and $H_2O_2$ 0.05% in 100mM pH 4.5 citrate buffer). Revelation is stopped by adding 50 $\mu$l/well HCl 1N. Optical densities are read at 490 and 620 nm by using Emax immunoreader (Molecular Devices). Antibody titre is calculated by the 4 parameter mathematical method using Soft-MaxPro software.

*Opsonophagocytosis assay*

The purpose of this assay is to reproducibly measure the opsonising capacity of test serum samples against *Streptococcus pneumoniae* serotypes 1, 3, 4, 5, 6B, 7F, 9V, 14, 18C, 19F or 23F using a method adapted from the published standardized method of the CDC (Steiner et al, Clinical and Diagnostic Laboratory Immunology 4: 415. 1997).

This assay reproduces *in vitro* what occurs *in vivo* as the primary mechanism of eliminating invading *Streptococcus pneumoniac* or pneumococci. That is opsonization of the pneumococci followed by phagocytosis and then killing. "Phagocytosis" is the process by which cells engulf material and enclose it within a vacuole (phagosome) in the cytoplasm. Pneumococci are killed when they are phagocytised by healthy mammalian phagocytes. "Opsonization" is the process by which phagocytosis is facilitated by the deposition of opsonins, e.g. antibody and complement, on the antigen.

There have been numerous opsonophagocytic assays reported in the literature. The standardized method of the CDC was tested in a multi-lab setting (Steiner et al, ICAAC, Sept 16- 20, 2000, Toronto). This latter assay was adapted at SB since it provided a basis for comparison to other laboratories, it used reagents and controls that are generally available, and it expressed the results as the titre (dilution) of serum able to facilitate killing of 50% of viable pneumococci, a unit that is commonly used for this type of assay. Indeed, it was shown that the adapted assay could generate results that correponded quite well with 4 other laboratories (Steiner et al, ICAAC, Sept 16- 20, 2000, Toronto).

The phagocytic cell used in the assay is the HL60 cell line, which originated from an individual with promyelocytic leukemia and was established as a continuous cell line by Collins et al. in 1977 (Nature 270: 347-9). This cell line is composed of undifferentiated hematopoietic cells, that is 85% blast cells and promyelocytes, 6% myelocytes and 9% differentiated cells. Polar compounds can induce the differentiation of the cells into at least two different lineages. N,N-dimethylformamide induces granulocytic differentiation which yield polymorphonuclear-like cells (44% myelocytes and metamyelocytes and 53% banded and segmented PMNs).

In Version A2 of the assay, the sera to be tested are heat-inactivated and 8 two-fold serial dilutions starting at 1/4 are made in 96-well microplates in HBSS medium containing 0.3% BSA. The final volume of diluted serum in each well is 25µl.

Four volumes of HL60 cells at $10^7$ cells /ml (5 or 6 days post differentiation with Dimethyl formamide), 2 volumes of *S.pneumoniae* bacteria (at the appropriate dilution) and 1 volume of baby rabbit complement are mixed just prior to use, and 25 µl of the mixture is added to each well of the 96-well microplate containing the diluted sera. For serotypes 1, and 6B, the amount of complement is increased to 12.5% final concentration, giving Version A3 of the assay.

After two hours incubation at 37°C under orbital shaking, the plate is put on ice in order to stop the opsonophagocytosis reaction.

An estimate is made of the the colony forming units (CFU) in each well by overnight incubation at 37°C. The "opsonic titre" (OT) is defined as the reciprocal dilution of the serum able to reduce by at least 50% the number of *S.pneumoniae* bacteria in the wells (ie., 50 % killing). The % killing is calculated by the following fomulae:

$$\% \, killing = (Mean \, CFU \, control \, wells - CFU \, sample) \, / \, Mean \, CFU \, control \, wells \, x \, 100$$

### *Pneumococcal intranasal challenge in OFI mice*

Seven week-old OF1 female mice are intranasally inoculated under anesthesia with 5.10' CFU of mouse-adapted *S. pneumoniae* serotype 2, 4 or 6B. Lungs are removed at 6 hours after challenge and homogenized (Ultramax, 24000 rpm, 4°C) in Todd Hewith Broth (THB, Gibco) medium. Serial 10-fold dilutions of lung homogenates are plated overnight at 37°C onto Petri dishes containing yeast extract-supplemented THB agar. Pneumococcal lung infection is determined as the number of CFU/mouse, expressed as logarithmic weighted-average. Detection limit is 2.14 log CFU/mouse.

**Example 2A** *3D-MPL adjuvant effect on anti-protein immune response*

In the present example, we can evaluate the impact of 3D-MPL adjuvantation on the immune response to the protein of the invention.

Groups of 10 female 6 week-old Balb/c mice are intramuscularly immunized at days 0, 14 and 21 with 1 µg protein contained in either A: AlPO4 100 µg; or B: AlPO4 100 µg + 5 µg 3D-MPL (3 de-O-acylated monophosphoryl lipid A, supplied by Ribi Immunochem). ELISA IgG is measured in post-III sera.

Whichever the antigen, best immune responses can be shown to be induced in animals vaccinated with 3D-MPL-supplemented formulations.

**Example 2B** *Beneficial impact of the addition of a protein of the invention +/- 3D-MPL adjuvant on the protective effectiveness of PD-conjugated 11-valent polysaccharide vaccine against pneumococcal lung colonization in OF1 mice intranasally challenged with serotype 2,4 or 6B.*

In the present example, we can evaluate the prophylactic efficacy of a vaccine containing the 11-valent polysaccharide-protein D conjugate, a protein of the invention and AIP04 + 3D-MPL adjuvants, compared to the classical AlPO4-adsorbed 11-valent polysaccharide-protein D conjugate formulation.

Groups of 12 female 4 week-old OF1 mice are immunized subcutaneously at days 0 and 14 with formulations containing A: 50 μg AlPO4; B: 0.1 μg PS/serotype of PD-conjugated 11-valent polysaccharide vaccine + 50 μg AlPO4; or C: 0.1 μg PS/serotype of PD-conjugated 11-valent polysaccharide vaccine + 10 μg protein of the invention + 50 μg AlPO4 + 5 μg 3D-MPL (supplied by Ribi Immunochem). Challenge is done at day 21 as described above.

As can be shown by this method, a significant protection is conferred by the 11-valent polysaccharide conjugate vaccine supplemented with the protein of the invention and adjuvanted with AlPO4 + MPL. On the contrary, no significant protection is observed in animals immunized with the 11-valent polysaccharide conjugate / AlPO4 formulation. This result can prove that the addition of the protein of the invention and 3D-MPL adjuvant enhances the effectiveness of the 11-valent polysaccharide conjugate vaccine against pneumonia.

**Example 2C,** *immune correlates of the protection showed in example 2B*

In order to establish the immune correlates of protection conferred in example 2B, by the 11-valent polysaccharide conjugate vaccine supplemented with a protein of the invention and 3D-MPL, pre-challenge serological antibody responses to polysaccharide 2, 4 or 6B, and the protein of the invention can be measured as described above.

Antibody titers are then compared to bacteria colony numbers measured in lungs of the corresponding animals collected at 6 hours post-challenge. $R^2$ are calculated on Log/Log linear regressions.

Calculated $R^2$ can show the absence of correlation between humoral immune responses and protection for both antigens. Anti-6B (or 2 or 4) antibody titers are not significantly different in the groups immunized with the 11-valent conjugate vaccine or with the same vaccine supplemented with the protein of the invention and 3D-MPL. Therefore, the protection improvement seen with formulation C is not solely due to a higher antibody response to polysaccharide 6B (or 2 or 4).

Taken together, the results can suggest that protection is not mediated by humoral immune responses alone, but rather also by a cell-mediated immunity induced by the protein antigen (preferably in the presence of 3D-MPL). This can give additional support to the addition of protein antigen(s) and potent adjuvant(s) in the pneumococcal polysaccharide conjugate vaccine, so as to coordinate both arms of the immune system for optimal protection.

**Example 3 - The Cooperation of both arms of the Immune System in mice actively immunised with a protein of the invention and passively immunised with antibodies against pneumococcal PS**

**Example 3A** - Find the Concentration of Passively Administered Anti-6B-Polysaccharide (anti-PS) Antibody Protecting Against Pneumonia

Method

Vaccine Groups: Four groups of 16 mice were passively immunised (i.p.) on day -1 with 100 μl of undiluted rat anti-polysaccharide antisera according to the groups detailed below. (total 64 mice)

| Group | Specificity | IgG Concentration in Antisera |
|---|---|---|
| G1 | α-PS -6B | 5 μg/ml. |
| G2 | α-PS -6B | 2 μg/ml. |
| G3 | α-PS -6B | 0.75 μg/ml. |
| G4 | Control | 0 μg/ml. |

Animals: 64 male CD-1 mice from Charles River, Canada, weighing approx 35g (approx 10 weeks old).

Anesthesia: Mice were anesthetized with isoflurane (3%) plus 02(1 L/min).

Organism: *S. pneumoniae* N1387 (serotype 6) was harvested from trypticase soy agar plates (TSA) supplemented with 5% horse blood and suspended in 6 ml of PBS. Immediately prior to infection, 1 ml bacterial suspension was diluted into 9 ml of cooled molten nutrient agar (BBL) and kept at 41°C. Mice received approx 6.0 log10 cfu/mouse in a volume 50 ul.

Infection: On day 0 mice were anesthetized as described above and infected with *S. pneumoniae* N1387 (50μl cooled bacterial suspension) by intra-bronchial instillation via non-surgical intra-tracheal intubation. This method was described by Woodnut and Berry (Antimicrob. Ag. Chemotherap. 43: 29 (1999)).

Samples: On day 3 post infection, 8 mice/group were sacrificed by CO2 overdose and lungs were excised and homogenized in 1 ml PBS. Tenfold serial dilutions were prepared in PBS to enumerate viable bacterial numbers. Samples were inoculated (20 μl) in triplicate onto TSA plates supplemented with 5% horse blood and incubated overnight at 37°C prior to evaluation. Further sets of mice were sacrificed on day 7 and sampled as above.

Results:

| IgG conc (ug/ml) in rat sera | Bacterial numbers (log 10 cfu/lungs) at days post infection | |
|---|---|---|
| | 3 | 8 |
| 5 | 6.7 $\pm$ 0.7 (1/7) | 7.2 $\pm$ 0.7 (5/8) |
| 2 | 6.5$\pm$ 0.7 (1/7) | 6.9$\pm$ 1.8 (4/7) |
| 0.75 | 7.7$\pm$0.5 (5/8) | 4.8 $\pm$ 1.4 (2/8) |
| 0 | 6.7$\pm$1.5 (3/6) | 6.3 $\pm$1.5 (3/9) |

Figures in parenthesis are numbers of animals that died prior to sample time.

Conclusion: In general, there was no significant difference in bacterial numbers isolated from any of the treatment groups. This indicates that no measurable protection was afforded by the anti-polysaccharide antibody at concentrations up to and including 5 $\mu$g/ml.

This is similar to what is observed in some human clinical trials, that is, anti-polysaccharide antibody is insufficient to protect against pneumococcal pneumonia in some populations.

**Example 3B-** Determine the protection from pneumonia afforded by active administration of a protein of the invention with or without adjuvant, and synergy with sub-optimal anti-PS antibody.

Method

Animals: 128 male CD-1 mice (6 weeks old at old at immunisation, 10 weeks old at infection) from Charles River, St. Constant, Quebec, Canada. Animals weighed approx 20 gm at 6 weeks and 38g at 10 weeks.

Immunisations: Six groups of 16 mice are immunised by subcutaneous injection on days -22 and -14 with 100 $\mu$l of vaccine as detailed below. (Total 128 mice). 3D-MPL is obtained from Ribi/Corixa.

On day -1, specific groups (see Table below) are immunised (i.p.100 $\mu$l) passively with a concentration of 4.26 $\mu$g/ml (4 ml of 5 $\mu$/lml + 1.3 ml of 2 $\mu$g/ml) mouse anti-polysaccharide antibody.

| Group | Injection Volume Active | Vaccine given days -22, -14 (Dosage $\mu$g) | Injection Volume Passive | Passive IgG (day-1) |
|---|---|---|---|---|
| 1-1 | 100 $\mu$l s.c. | Protein/AlPO4 (10/50) | | None |
| 1-2 | 100 $\mu$l s.c. | Protein/MPUAlPO4 (10/5/50) | | None |
| 1-3 | 100 $\mu$l s.c. | Protein/AlPO4 (10/50) | 100 $\mu$l i.p. | $\alpha$-PS |
| 1-4 | 100 $\mu$l s.c. | Protein/MPL/AlPO4 (10/5/50) | 100 $\mu$l i.p. | $\alpha$-PS |
| 1-5 | 100 $\mu$l s.c. | MPL/AlPO4 (5/50) | 100 $\mu$l i.p. | $\alpha$-PS |
| 1-6 | 100 $\mu$l s.c. | MPL/AlPO4 (5/50) | | None |

Infection: On day 0, mice are anesthetized (3% isoflurane plus 1 L/min $O_2$), Bacterial inocula are prepared by harvesting growth of *S. pneumoniae* N1387 (serotype 6) from trypticase soy agar plates (TSA) supplemented with 5% horse blood and suspending in 6 ml of PBS. A ten-fold dilution (1ml plus 9ml) is prepared in cooled molten nutrient agar (kept at 41°C) immediately prior to infection. Mice are infected by intra-bronchial instillation via intra-tracheal intubation and receive approximately 6.0 log10 cfu/mouse in a volume of 50 $\mu$l. This method was described by Woodnut and Berry (Antimicrob. Ag. Chemotherap. 43: 29 (1999)).

Samples: At 72 post infection, 8 mice/group are sacrificed by $CO_2$ overdose and the lungs are excised and homogenized in 1 ml PBS. Tenfold serial dilutions are prepared in PBS to enumerate viable bacterial numbers. Samples are inoculated (20 $\mu$l) in triplicate onto TSA plates supplemented with 5% horse blood and incubated overnight 37°C prior to evaluation. Further sets of mice are sacrificed on day 8 post-infection and samples as above.

Analysis of Data

The outcome measure for comparison of treatment is the number of bacteria in the lungs at 3 and 7 day post infection. Results can be presented as group means with standard deviations. Statistical analysis should be performed using the Students t-test where a P value of <0.05 is considered significant.

As demonstrated above, anti-polysaccharide antibody alone (Group 1-5) can be shown not to afford protection against growth of pneumococci in the lung. Pneumococcal protein adjuvanted with AlPO4 (Group 1-1) may not confer protection either, but will do to a better extent when Protein is combined with 3D-MPL (Group 1-2).

Most significant protection can be seen in groups with both anti-polysaccharide antibody and protein, particularly in the group having all three elements, Protein, 3D-MPL and passively administered anti-polysaccharide antibody (Group 1-4). This conclusion can also be supported by the mortality rate. Groups 1-3 and, particularly, 1-4 will have fewer deaths compared to the other groups.

Conclusion:

As the experiment is done with passively immunised animals, the synergistic effect of also actively immunising with protein (+/- MPL) cannot be due to an increase in the level of antibodies against the polysaccharide antigen.

Significant protection against pneumococcal pneumonia can be seen in groups immunised with both protein plus passively administered anti-polysaccharide antibody, particularly if 3D-MPL is also present, indicating the synergy of this combination.

If the anti-polysaccharide immunisation is carried out actively (preferably with conjugated polysaccharide), this effect will be even more marked, as the effect of B-cell memory, and constant levels of anti-PS antibody throughout the experiment will contribute to the immune response cooperation.

**Example 4 - Method to determine synergy by correlate of protection**

The principle mechanism of protection that the human body uses to eliminate infecting pneumococcus is antibody mediated opsonophagocytosis (Bruyn et al. Clin. Infect Dis. 14: 251 (1992)). Whereas several ELISA methods have been developed to measure the antibody concentration to capsular polysaccharide as a correlate of protection, it has become apparent that the *in vitro* opsonophagocytosis assay is a better correlate of protection (Musher et al. J. Infect Dis. 182: 158 (2000)).

The Pneumococcal proteins of the invention provide protection against pneumococcal infection by mechanisms that are different from antibody mediated opsonophagocytosis. In example 2, active immunisation with both conjugate and protein can show a synergic effect, which can not be explained by the antibody concentration differences since they are the same in both groups. Thus the residual protection that can be observed must come from a synergistic effect. Similarly, since the antibody is added passively, the same conclusion can be reached in example 3.

In many cases, the pneumococcal proteins of the invention are surface associated, and are expected to provide some opsonic activity themselves. In this case it is possible to distinguish the mechanism of protection via a quantitative measure of the opsonising capacity of the the anti-pneumococcal protein, which can be used to estimate the relative contribution of opsonic activity to other synergeic mechanisms of protection.

In the mouse lung colonisation model, the relative protection of each vaccine can be estimated from the clearance of bacteria from the lungs. Or alternatively, the vaccine efficacy can be estimated from case rates, as normally determined for vaccines.

**% Protection = (CFU/lung Control – CFU/lung Vaccine)/(CFU/lung Control)**

$$\% \text{ Efficacy} = (\text{Cases Control} - \text{Cases Vaccine})/(\text{Cases Control})$$

To determine the portion of the protection or efficacy that originates from the synergistic effect, it is a matter of determining which portion of the efficacy would be expected based on the ratio of the opsonic titres.

In Example 3 above, the % protection by the combination is due to synergy between the protein/antibody components as neither the protein nor the anti-polysacharide antibody alone can provide much protection themselves.

It is possible to estimate the amount of protection of the synergeic effect on the basis of the relative opsonic activity. If the opsonic activity afforded by a anti-capsular polysacharide antibody is X, and the opsonic activity afforded by anti-pneumococcal protein antibody is Y, then the total opsonic activity can be shown to be X + Y, and the relative portion of the opsonic activity of the protein would be Y/X+Y. This is compared to the relative protective efficacy of a vaccine, where the anti-polysaccharide portion of the vaccine provides a protective efficacy of A%, and the protective efficacy of the vaccine of polysaccharide plus protein is B%. The additional efficacy that can not be accounted by opsonic acitivity is then estimated as

$$\text{Residual Protective Activity (Synergy)} = B\% - A\% - B\%* (Y/X+Y)$$

This example is not intended to limit the ways to estimate the effect of synergy. Once other correlates of protection have been identified, they could be used to estimate this synergisic effect.

## Claims

1. An immunogenic composition comprising at least one *Streptococcus pneumoniae* polysaccharide antigen and at least one *Streptococcus pneumoniae* protein antigen selected from the group consisting of: PhtA, PhtD, PhtB and PhtE or immunologically functional equivalent thereof.

2. The immunogenic composition of claim 1 wherein the *Streptococcus pneumoniae* protein antigen is PhtD.

3. The immunogenic composition of claim 2 further comprising Ply.

4. The immunogenic composition a s claimed in any one of claims 1-3, wherein the polysaccharide antigen is presented in the form of a polysaccharide-protein carrier conjugate.

5. The immunogenic composition of claim 4, wherein the carrier protein is selected from the group consisting of: Diphtheria toxoid, Tetanus toxoid, CRM197, Keyhole Limpet Haemocyanin (KLH), protein derivative of Tuberculin (PPD), and protein D from *H. influenzae.*

6. An immunogenic composition as claimed in any of claims 1 to 5 wherein the vaccine comprises at least four pneumococcal polysaccharide antigens from different serotypes.

7. An immunogenic composition as claimed herein additionally comprising an adjuvant.

8. An immunogenic composition as claimed in claim 7, wherein the adjuvant comprises an aluminium salt.

9. An immunogenic composition as claimed in claim 7, wherein the adjuvant is a preferential inducer of a TH1 response.

10. An immunogenic composition as claimed in claim 9, wherein the adjuvant comprises at least one of the following: 3D-MPL, a saponin immunostimulant, or an immunostimulatory CpG oligonucleotide.

11. An immunogenic composition as claimed in claim 10, wherein the adjuvant comprises a carrier selected from the group comprising: an oil in water emulsion, liposomes, and an aluminium salt.

12. An immunogenic composition as claimed herein for use as a medicament.

13. A vaccine comprising the immunogenic composition of claims 1-11.

**14.** Use of a pneumococcal polysaccharide antigen in combination with a *Streptoccocus pneumoniae* protein antigen selected from the group consisting of PhtA, PhtD, PhtB and PhtE, and optionally a TH1 inducing adjuvant, in the manufacture of a medicament for the prevention or treatment of pneumonia in patients over 55 years.

**15.** Use of a pneumococcal polysaccharide antigen in combination with a *Streptoccocus pneumoniae* protein antigen selected from the group consisting of PhtA, PhtD, PhtB and PhtE, and optionally a TH1 inducing adjuvant, in the manufacture of a medicament for the prevention or treatment of otitis media in infants or toddlers.

**16.** A method of making an immunogenic composition as claimed herein, comprising the steps of:

> selecting one or more pneumococcal polysaccharide antigen(s);
> selecting one or more pneumococcal protein antigen(s) from the group consisting of PhtA, PhtD, PhtB and PhtE; and
> mixing said polysaccharide and protein antigens with a suitable excipient.


**Patentansprüche**

**1.** Immunogene Zusammensetzung, umfassend mindestens ein Streptococcus pneumoniae-Polysaccharidantigen und mindestens ein Streptococcus pneumoniae-Proteinantigen ausgewählt aus der Gruppe bestehend aus: PhtA, PhtD, PhtB und PhtE oder einem immunologischen funktionellen Äquivalent davon.

**2.** Immunogene Zusammensetzung gemäß Anspruch 1, wobei das Streptococcus pneumoniae-Proteinantigen PhtD ist.

**3.** Immunogene Zusammensetzung gemäß Anspruch 2, weiter umfassend Ply.

**4.** Immunogene Zusammensetzung gemäß irgendeinem der Ansprüche 1 bis 3, wobei das Polysaccharidantigen in Form eines Polysaccharid-Protein-Trägerkonjugats vorliegt.

**5.** Immunogene Zusammensetzung gemäß Anspruch 4, wobei das Trägerprotein aus der Gruppe bestehend aus:

> Diphtherietoxoid, Tetanustoxoid, CRM197, Keyhole Limpet Haemocyanin (KLH), Proteinderivat von Tuberculin (PPD) und Protein D von H. influenzae ausgewählt ist.

**6.** Immunogene Zusammensetzung gemäß irgendeinem der Ansprüche 1 bis 5, wobei der Impfstoff mindestens vier Pneumococcen-Polysaccharidantigene verschiedener Serotypen umfasst.

**7.** Immunogene Zusammensetzung, wie hier beansprucht, zusätzlich umfassend ein Adjuvans.

**8.** Immunogene Zusammensetzung gemäß Anspruch 7, wobei das Adjuvans ein Aluminiumsalz umfasst.

**9.** Immunogene Zusammensetzung gemäß Anspruch 7, wobei das Adjuvans ein bevorzugter Induktor einer TH1-Antwort ist.

**10.** Immunogene Zusammensetzung gemäß Anspruch 9, wobei das Adjuvans mindestens einen der folgenden umfasst: 3D-MPL, eine Saponin-Immunstimulanz oder ein immunstimulierendes CpG-Oligonukleotid.

**11.** Immunogene Zusammensetzung gemäß Anspruch 10, wobei das Adjuvans weiter einen Träger umfasst, der aus der Gruppe ausgewählt ist, die umfasst: eine Öl-in-Wasser-Emulsion, Liposomen und ein Aluminiumsalz.

**12.** Immunogene Zusammensetzung, wie hier beansprucht, zur Verwendung als Medikament.

**13.** Impfstoff umfassend die immunogene Zusammensetzung der Ansprüche 1 bis 11.

**14.** Verwendung eines Pneumococcen-Polysaccharidantigens in Kombination mit einem Streptococcus pneumoniae-Proteinantigen ausgewählt aus der Gruppe bestehend aus PhtA, PhtD, PhtB und PhtE und ggf. ein TH1-induzierendes Adjuvans in der Herstellung eines Medikaments zur Prävention oder Behandlung von Pneumonie in Patienten über 55 Jahren.

**15.** Verwendung eines Pneumococcen-Polysaccharidantigens in Kombination mit einem Streptococcus pneumoniae-Proteinantigen ausgewählt aus der Gruppe bestehend aus PhtA, PhtD, PhtB und PhtE und ggf. einem TH1-induzierenden Adjuvans in der Herstellung eines Medikaments zur Prävention oder Behandlung von Otitis media in Kleinkindern oder Säuglingen.

**16.** Verfahren zur Herstellung einer immunognen Zusammensetzung wie hier beansprucht, umfassend die Schritte:

Auswählen eines oder mehrerer Pneumococcen-Polysaccharidantigene;
Auswählen eines oder mehrerer Pneumococcen-Proteinantigene aus der Gruppe bestehend aus PhtA, PhtD, PhtB und PhtE; und
Mischen dieser Polysaccharid- und Proteinantigene mit einem geeigneten Exzipienten.

**Revendications**

**1.** Composition immunogène comprenant au moins un antigène polysaccharidique de *Streptococcus pneumoniae* et au moins un antigène protéique de *Streptococcus pneumoniae* choisi dans le groupe constitué par: PhtA, PhtD, PhtB et PhtE ou leurs équivalents fonctionnels sur le plan immunologique.

**2.** Composition immunogène selon la revendication 1, dans laquelle l'antigène protéique de *Streptococcus pneumoniae* est PhtD.

**3.** Composition immunogène selon la revendication 2, comprenant en outre Plv.

**4.** Composition immunogène selon l'une quelconque des revendications 1 à 3, dans laquelle l'antigène polysaccharidique se présente sous la forme d'un conjugué polysaccharide-support protéique.

**5.** Composition immunogène selon la revendication 4, dans laquelle la protéine support est choisie dans le groupe constitué par: l'anatoxine diphtérique, l'anatoxine tétanique, le CRM197, l'hémocyanine de patelle (KLH), un dérivé protéique de la tuberculine (PPD) et la protéine D de *H. influenzae.*

**6.** Composition immunogène selon l'une quelconque des revendications 1 à 5, dans laquelle le vaccin comprend au moins quatre antigènes polysaccharidiques pneumococciques provenant de différents sérotypes.

**7.** Composition immunogène selon les présentes revendications, comprenant en outre un adjuvant.

**8.** Composition immunogène selon la revendication 7, dans laquelle l'adjuvant comprend un sel d'aluminium.

**9.** Composition immunogène selon la revendication 7, dans laquelle l'adjuvant est un inducteur préférentiel de réponse TH1.

**10.** Composition immunogène selon la revendication 9, dans laquelle l'adjuvant comprend au moins l'un des éléments suivants: 3D-MPL, un immunostimulant à base de saponine ou un oligonucléotide CpG immuno-stimulatoire.

**11.** Composition immunogène selon la revendication 10, dans laquelle l'adjuvant comprend un support choisi dans le groupe comprenant: une émulsion huile dans l'eau, des liposomes et un sel d'aluminium.

**12.** Composition immunogène selon les selon les présentes revendications, utilisable comme médicament.

**13.** Vaccin comprenant la composition immunogène selon les revendications 1 à 11.

**14.** Utilisation d'un antigène polysaccharidique pneumococcique en combinaison avec un antigène protéique de *Streptococcus pneumoniae* choisi dans le groupe constitué par PhtA, PhtD, PhtB et PhtE, et facultativement un adjuvant induisant TH1, dans la préparation d'un médicament destiné à la prévention ou au traitement de la pneumonie chez des patients de plus de 55 ans.

**15.** Utilisation d'un antigène polysaccharidique pneumococcique en combinaison avec un antigène protéique de *Streptococcus pneumoniae* choisi dans le groupe constitué par PhtA, PhtD, PhtB et PhtE, et facultativement un adjuvant induisant TH1, dans la préparation d'un médicament destiné à la prévention ou au traitement de l'otite moyenne chez les nourrissons ou les jeunes enfants.

**16.** Procédé de préparation d'une composition immunogène selon les selon les présentes revendications, comprenant les étapes suivantes:

- choisir un ou plusieurs antigène(s) polysaccharidique(s) pneumococcique(s);
- choisir un ou plusieurs antigène(s) protéique(s) pneumococcique(s) dans le groupe constitué par PhtA, PhtD, PhtB et PhtE ; et
- mélanger lesdits antigènes polysaccharidiques et protéiques avec un excipient approprié.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 497524 A
- EP 497525 A
- WO 9818930 A
- WO 0037105 A
- WO 0017370 A
- WO 99115675 A
- WO 0030299 A
- WO 9605859 A
- WO 9006951 A
- WO 9903884 A
- US 5804193 A
- WO 9214488 A
- WO 9953940 A
- WO 9709994 A
- WO 9741151 A
- WO 9951266 A
- WO 9640928 A
- EP 0837130 A
- EP 0834568 A
- WO 0029434 A
- WO 9821337 A
- WO 9951188 A
- EP 594610 B
- US 4372945 A, Likhite
- US 4474757 A, Armor
- WO 9508348 A
- GB 2220211 A
- EP 689454 B1
- WO 9400153 A
- WO 9633739 A
- WO 9517210 A
- WO 9602555 A
- US 4235877 A
- WO 9934850 A
- EP 1092444 A
- WO 0113977 A
- US 5480381 A
- US 5599302 A
- US 5334144 A
- US 5993412 A
- US 5649912 A
- US 5569189 A
- US 5704911 A
- US 5383851 A
- US 5893397 A
- US 5466220 A
- US 5339163 A
- US 5312335 A
- US 5503627 A
- US 5064413 A
- US 5520 A
- US 639 A
- US 4596556 A
- US 4790824 A
- US 4941880 A
- US 4940460 A
- WO 97137705 A
- WO 9713537 A
- WO 9927961 A
- WO 9748440 A
- WO 9828037 A
- WO 9820734 A
- WO 9741731 A
- WO 9634960 A
- WO 9855606 A
- WO 9713785 A
- WO 9732980 A
- WO 9303761 A
- EP 9903824 W
- EP 9903823 W
- EP 0001468 W
- GB 9917977 A
- GB 9918208 A
- GB 9918302 A
- GB 9918038 A
- EP 9903038 W
- EP 9903822 W
- EP 9906781 W
- EP 9903257 W
- US 5766608 A
- US 5843464 A
- WO 9964067 A
- WO 9701638 A
- EP 281673 A
- WO 9412641 A
- EP 594610 A
- WO 9426304 A
- EP 72513 A

**Non-patent literature cited in the description**

- **Peltola et al.** *N. Engl. J. Med.,* 1984, vol. 310, 1561-1566

- **Roghmann et al.** *J. Gerontol.,* 1987, vol. 42, 265-270
- **Verghese ; Berk.** *Medicine (Baltimore),* 1983, vol. 62, 271-285
- **Fedson et al.** *Arch Intern Med.,* 1994, vol. 154, 2531-2535
- **Fine et al.** *Arch Intern Med.,* 1994, vol. 154, 2666-2677
- **Jameson ; Wolf.** *CABIOS,* 1988, vol. 4, 181-186
- **Berry ; Paton.** *Infect Immun,* December 1996, vol. 64 (12), 5255-62
- **Sanchez-Beato et al.** *FEMS Microbiol Lett,* 1998, vol. 164, 207-14
- **Mosmann, T.R. ; Coffman, R.L.** TH1 and TH2 cells: different patterns of lymphokine secretion lead to different functional properties. *Annual Review of Immunology,* 1989, vol. 7, 145-173
- **Thoelen et al.** *Vaccine,* 1998, vol. 16, 708-14
- The subunit and adjuvant approach. Vaccine Design. Plenum Press, 1995
- **Helminen ME et al.** *Infect. Immun.,* 1993, vol. 61, 2003-2010
- **Chaloupka I. et al.** *Eur. Journal Clin. Microbiol. Infect. Dis.,* 1996, vol. 15, 121-127
- **Schmidt A. C. et al.** *J Virol,* May 2001, 4594-4603
- **Janson et al.** *Infect. Immun.,* 1991, vol. 59, 119-125
- **Steiner et al.** *Clinical and Diagnostic Laboratory Immunology,* 1997, vol. 4, 415
- **Steiner et al.** *ICAAC,* 16 September 2000
- **Steiner et al.** *ICAAC,* 20 September 2000
- **Collins et al.** *Nature,* 1977, vol. 270, 347-9
- **Woodnut ; Berry.** *Antimicrob. Ag. Chemotherap.,* 1999, vol. 43, 29
- **Bruyn et al.** *Clin. Infect Dis.,* 1992, vol. 14, 251
- **Musher et al.** *J. Infect Dis.,* 2000, vol. 182, 158